(19) Europäisches Patentamt

European Patent Office

Office européen des brevets

(11) Veröffentlichungsnummer: **0 457 035 B1**

(12) **EUROPÄISCHE PATENTSCHRIFT**

(45) Veröffentlichungstag der Patentschrift: **02.08.95**

(51) Int. Cl.⁶: **A61L 2/04**, C12N 1/08

(21) Anmeldenummer: **91105898.0**

(22) Anmeldetag: **13.04.91**

(54) Verfahren zur Inaktivierung der biologischen Aktivität von DNA.

(30) Priorität: **17.05.90 DE 4015832**

(43) Veröffentlichungstag der Anmeldung:
**21.11.91 Patentblatt 91/47**

(45) Bekanntmachung des Hinweises auf die
Patenterteilung:
**02.08.95 Patentblatt 95/31**

(84) Benannte Vertragsstaaten:
**AT BE CH DE DK ES FR GB GR IT LI LU NL SE**

(56) Entgegenhaltungen:
**EP-A- 0 144 714**
**EP-A- 0 278 674**
**FR-A- 2 318 225**
**GB-A- 1 471 336**
**US-A- 3 227 626**

(73) Patentinhaber: **Dr. Karl Thomae GmbH**

**D-88397 Biberach (DE)**

(72) Erfinder: **Bergemann, Klaus, Dr. Dipl.-Chem.**
**Hugo-Häring-Strasse 49**
**W-7950 Biberach 1 (DE)**
Erfinder: **Bader, Georg**
**Am Schlegelberg 16**
**W-7951 Birkenhard (DE)**
Erfinder: **Berthold, Wolfgang, Dr.**
**Friedrich-Ebert-Strasse 32**
**W-7950 Biberach 1 (DE)**
Erfinder: **Werner, Rolf-Günter, Prof. Dr.**
**Dipl.-Mikrobiologe**
**Hugo-Häring-Strasse 72**
**W-7950 Biberach 1 (DE)**

## Beschreibung

Die Erfindung betrifft ein Verfahren zur Inaktivierung der biologischen Aktivität von DNA, insbesondere rekombinanter DNA, und vorzugsweise die gleichzeitige Abtötung von Zellen wie Säugetierzellen, z.B. CHO-, COS- und Hybridoma-Zellen, Bakterienzellen wie z.B. E.coli und B.subtilis, oder Hefen.

Beim Arbeiten mit rekombinanter DNA enthaltenden Mikroorganismen entstehen Abfälle, die nicht nur lebende Organismen sondern auch aktive Nukleinsäure enthaltende tote Organismen enthalten. Die Abfälle müssen bei entsprechender Sicherheitseinstufung inaktiviert werden, bevor die Biomasse in die Kläranlage eingeleitet wird.

Obwohl viele Mikroorganismen und Zellkulturen durch Erhitzung auf 80°C abgetötet werden können, wird die DNA durch diese Maßnahmen in der Regel nicht zerstört.

Die bisher bekannten und vom Bundesgesundheitsamt (BGA) der Bundesrepublik Deutschland und von der Zentralkommission für Biologische Sicherheit (ZKBS) der Bundesrepublik Deutschland anerkannten Methoden zur Sterilisation und Inaktivierung (z.B. Dampfsterilisation 20 Minuten lang bei 121°C) sind sehr aufwendig, und im Industriemaßstab angewandt, sehr kostenintensiv. Bei diesen von der ZKBS zugelassenen Entsorgungsverfahren wird der Abbau der DNA auf kleine Bruchstücke, unter 500 bp, erreicht. Derartige Bruchstücke sind nicht mehr biologisch aktiv und können daher gefahrlos in das Abwasser geleitet werden.

Da die Inaktivierung erfolgen muß, bevor die Biomasse in die Kläranlage eingeleitet wird, können keine Substanzen zu diesem Zweck verwendet werden, die beispielsweise die Mikroorganismen des Belebtschlammes schädigen oder abtöten könnten, falls eine biologische Kläranlage nach dem Belebtschlammverfahren beschickt wird. Darüber hinaus ist es erstrebenswert, daß die verwendeten Substanzen für die Umwelt nicht toxisch sind bzw. von den Mikroorganismen des Belebtschlammes abgebaut werden, damit sie keinen Schaden verursachen können und daß Substanzen verwendet werden, die die Oberflächen und Wandungen von Geräten, die für Fermentation und Aufreinigung eingesetzt werden, nicht negativ beeinflussen.

In der DE OS 37 33921 ist ein Verfahren zur Inaktivierung der DNA beschrieben, insbesondere rekombinanter DNA, wobei einer DNA enthaltenden Biomasse Percarbonsäure mit 1 bis 3 C-Atomen, eines ihrer Salze, ein Alkaliperoxid oder ein Alkaliperoxomonosulfat zugesetzt und die Mischung anschliessend 20 bis 60 Minuten lang auf 60 bis 100 °C erhitzt wird, vorzugsweise bei pH 6 bis 11.

Die Percarbonsäure, wie z.B. die Peroxiessigsäure, sind aber relativ instabil.

Es ist bereits literaturbekannt, daß eine DNA-Kette bei einer 40 Minuten langen Erhitzung in einer Phenol enthaltenden Kochsalzlösung bei 100°C stark degradiert wird (Meth. Enzymol. B XII, S. 97).

In Meth. Enzymol. A XII, S. 222-223, wird die Degradierung einer DNA zur Herstellung von Apurinsäure mittels einer 98%igen Ameisensäure bei 30°C für 17 Stunden beschrieben.

In der FR-A-2 318 225 wird ein Verfahren zur Reduktion des Nukleinsäuregehaltes von Mikroorganismenzellen ohne mechanische Fragmentierung der Zellen beschrieben, in dem zunächst eine Behandlung mit einer Säure zur Einstellung eines pH-Wertes von nicht größer als 5 erfolgt, anschließend oder gleichzeitig auf eine Temperatur von mindestens 60°C erhitzt wird und danach eine Behandlung mit einer Base zur Erhöhung des pH-Wertes auf mindestens 6 durchgeführt wird. Als verwendbare Säuren werden anorganische Säuren wie Schwefelsäure, Salzsäure oder Phosphorsäure sowie saure Gase wie Kohlendioxid oder Schwefeldioxid genannt.

Überraschenderweise haben wir gefunden, daß eine Reihe von lagerstabilen, biologisch abbaubaren Säuren bei gewissen pH-Werten und Konzentrationen nicht nur zur Inaktivierung von DNA sondern auch zur Abtötung von Zellen geeignet sind, und zwar unter relativ niedrigen Temperaturen.

Gegenstand der Erfindung ist daher ein Verfahren zur Inaktivierung der biologischen Aktivität der DNA, insbesondere der rekombinanten DNA, wobei ein Abbau der DNA erfolgt, dadurch gekennzeichnet, daß die DNA in Anwesenheit einer biologisch abbaubaren Säure ausgewählt aus Ameisensäure, Essigsäure, Propionsäure, Oxalsäure, Malonsäure, Bernsteinsäure, Äpfelsäure, Zitronensäure, Weinsäure, Milchsäure, Maleinsäure und Fumarsäure sowie geeigneter Kombinationen davon bei einer Konzentration von mindestens 0,2 mM und einem pH-Wert von maximal 4 während einer Dauer von 10 bis 70 Minuten auf eine Temperatur von 60 bis 100°C erhitzt wird.

Das Verfahren kann mit einer Biomasse oder isolierter DNA durchgeführt werden. Die Biomasse kann beispielsweise eine Mikroorganismen enthaltende Kulturlösung oder eine Zellkultur sein.

Bevorzugt wird ein Verfahren in Säure in einer Konzentration von 1 mM bis 500 mM, z.B. 2 bis 100 mM, insbesondere 2,3 bis 50 oder 10 bis 50 mM.

Das erfindungsgemäße Verfahren wird normalerweise im pH-Bereich zwischen 1 und 3, besonders bevorzugt im pH-Bereich zwischen 2 und 3, durchgeführt.

Bevorzugt werden Säugetierzellen bei einer Konzentration von mindestens 100 mM und einem pH-Wert von maximal 3, vorzugsweise 2.5, behandelt. Im Falle von Bakterien werden diese vorzugsweise bei einer Konzentration von mindestens 400 mM und einem pH-Wert von maximal 3 behandelt.

Die Erhitzung der Mischung erfolgt bevorzugt so lange, bis elektrophoretisch keine DNA mit einer Kettenlänge von mehr als 500 Basenpaaren nachweisbar ist. In der Regel beträgt die Dauer 10 bis 70 Minuten, insbesondere 20 bis 60 Minuten.

Um zu überprüfen, ob die gesamte DNA abgebaut wurde, wird aus der Mischung ein Aliquot entnommen und damit eine Agarosegel-Elektrophorese durchgeführt. Die chromatographische Auftrennung nach Molekulargewicht zeigt die Bruchstücke an, die noch in der Lösung vorhanden sind.

Ein besonderer Vorteil des Verfahrens besteht darin, daß nicht nur normal-doppelsträngige DNA wie λ DNA und genomische DNA aus Bakterien oder Säugetierzellen, sondern auch "supercoiled-Plasmid-DNA" sicher inaktiviert werden können.

BEISPIEL 1

Eine Probe, enthaltend 5 μg DNA in einer wässrigen Lösung (10 μl), wurde in einem 1.5 ml-Eppendorf-Gefäß angesetzt. Dazu wurde in verschiedenen Ansätzen (10 μl) Säure zugesetzt und die resultierende Reaktionsmischung (Endvolumen betrug 20 μl) unter den in der nachfolgenden Tabelle 1 jeweils angegebenen Bedingungen inkubiert. Am Ende der Inkubationszeit wurden 5 μl lM Tris pH 8,5 zur Neutralisation zugesetzt.

10 μl der DNA-Lösung wurden nach der Säurebehandlung entnommen, mit 5 μl Auftragspuffer, bestehend aus 20% Ficoll (Pharmacia Fine Chemicals), 0,05% Bromphenolblau, 20 mM EDTA, vermischt und auf eine Agarosegel-Elektrophoreseplatte (Agarose-Konzentrationen zwischen 1 und 2%) aufgetragen. Der Elektrophoresepuffer bestand aus 40 mM Tris-Acetat pH 7,8 1 mM EDTA. Die Elektrophoresen wurden bei 30 - 50 Volt/cm durchgeführt.

Als DNA-Längenstandards dienten λ-DNA verdaut mit Bst EII, oder Hind III und Plasmid-pBR 322 verdaut mit Hinf I oder Bst NI. Dabei wurden Fragmente mit folgenden Längen (in Basenpaaren) erhalten:

λ-DNA/Hind III:  27491, 9416, 6557, 4361, 2322, 2027, 564, 125
pBR 322/Bst NI:  1857, 1060, 929, 383, 121, 13

EP 0 457 035 B1

Tabelle 1

| DNA oder Zellen | Säure | Konz. | pH | Temp. | Inkuba-tions-zeit | Ergebnis |
|---|---|---|---|---|---|---|
| λ-DNA 48.500 Bp Boehringer Mannheim | Zitronens. | 3mM | 3.1 | 70°C | 10 bis 30 Min. | Totalabbau zu Fragmen-ten <500 bp |
| " | " | 200mM | 1.9 | 70°C | 10 Min. | " |
| " | " | 10mM | 2.8 | 70°C | 60 Min. | " |
| " | " | 1mM | 3.6 | 70°C | 60 Min. | " |
| " | " | 2,3mM | 3.1 | 60°C | 60 Min. | " |
| CHO-Zellen (96% lebende) | " | 50mM | 2.3 | 70°C | 60 Min. | " |
| " | " | 100mM | 2.1 | 70°C | 60 Min. | " |
| " | " | 200mM | 1.9 | 70°C | 60 Min. | " |
| " | " | 500mM | 1.7 | 70°C | 60 Min. | " |

| DNA oder Zellen | Säure | Konz. | pH | Temp. | Inkubationszeit | Ergebnis | |
|---|---|---|---|---|---|---|---|
| E. coli HB 101 | " | 100mM | 2,1 | 70°C | 60 Min. | keine lebensfähigen Zellen | (Bei Kultivierung auf Agarplatten und in Flüssigkulturen über 7 Tage bei 37°C) |
| " " | " | 200mM | 1.9 | 70°C | 60 Min. | " | |
| " " | " | 400mM | 1.8 | 70°C | 60 Min. | keine lebensfähigen Zellen und Totalabbau der DNA zu Fragmenten ‹ 500bp | " |
| " | " | 800mM | 1.5 | 70°C | 60 Min. | " | |
| " | " | 1000mM | 1.4 | 70°C | 60 Min. | " | |

EP 0 457 035 B1

| DNA oder Zellen | Säure | Konz. | pH | Temp. | Inkuba- tionszeit | Ergebnis |
|---|---|---|---|---|---|---|
| Plasmid DNA (pBR 322) (supercoiled 4.300 bp) | Ameisens. | 10mM | 3.6 | 70°C | 60 Min. | Totalabbau zu Fragmen- ten < 500 bp |
| " | " | 1mM | 4.0 | 70°C | 60 Min. | " |
| " | " | 1mM | 3.3 | 70°C | 60 Min.. | " |
| " | Zitronens. | 2,3mM | 3.1 | 60°C | 60 Min. | " |

BEISPIEL 2

Beispiel 1 wurde unter Verwendung von pBR 322 (supercoiled DNA) mit verschiedenen Säuren unterschiedlicher Konzentrationen, wie in Tabelle 2 dargestellt, wiederholt.

Die Reaktionsmischung wurde 60 Minuten lang bei 70°C inkubiert.

Tabelle 2

| Lane | | | pH |
|---|---|---|---|
| 1 | Zitronensäure | 10 mM | 2.8 |
| 2 | Zitronensäure | 1 mM | 3.6 |
| 3 | Zitronensäure | 0,1 mM | 4.3 |
| 4 | Essigsäure | 10 mM | 3.6 |
| 5 | Essigsäure | 1 mM | 4.1 |
| 6 | Essigsäure | 0,1 mM | 4.4 |
| 7 | Salzsäure | 10 mM | 2.3 |
| 8 | Salzsäure | 1 mM | 3.3 |
| 9 | Salzsäure | 0,1 mM | 4.1 |
| 10 | Phosphorsäure | 3 mM | 2.4 |
| 11 | Phosphorsäure | 0,3 mM | 3.1 |
| 12 | Phosphorsäure | 0,03 mM | 4.0 |
| 13 | ohne Säure | | |
| 14 | Längenstandard: pBR 322/Bst NI | | |

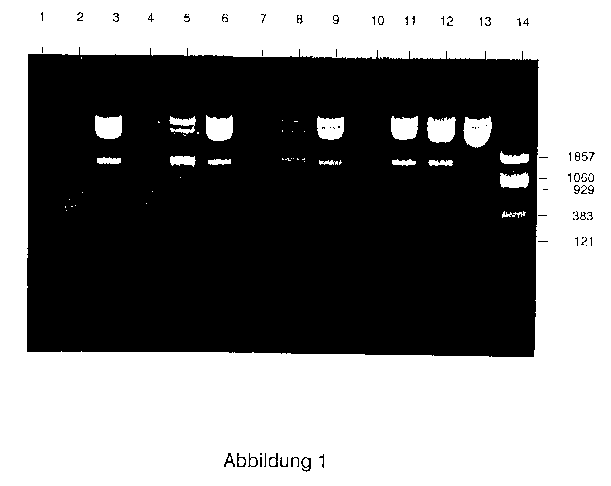

Abbildung 1 stellt die Elektrophorese eines solchen Versuches dar. Sie zeigt deutlich, daß bei erfindungsgemässer Inkubation der DNA (Lanes 1, 2, 4, 5, 7, 8 und 10) ein Abbau zu Fragmenten von kleiner als 300 bp erfolgt.

BEISPIEL 3

Beispiel 1 wurde unter Verwendung von Lambda-Phagen DNA mit verschiedenen Säuren unterschiedlicher Konzentrationen wie in Tabelle 3 dargestellt wiederholt.

Die Reaktionsmischung wurde 60 Minuten lang bei 70 °C inkubiert.

Tabelle 3

| Lane | | | pH |
|---|---|---|---|
| 1 | Zitronensäure | 10 mM | 2.8 |
| 2 | Zitronensäure | 1 mM | 3.6 |
| 3 | Zitronensäure | 0,1 mM | 4.3 |
| 4 | Essigsäure | 10 mM | 3.6 |
| 5 | Essigsäure | 1 mM | 4.1 |
| 6 | Essigsäure | 0,1 mM | 4.4 |
| 7 | Salzsäure | 10 mM | 2.3 |
| 8 | Salzsäure | 1 mM | 3.3 |
| 9 | Salzsäure | 0,1 mM | 4.1 |
| 10 | Phosphorsäure | 3 mM | 2.4 |
| 11 | Phosphorsäure | 0,3 mM | 3.1 |
| 12 | Phosphorsäure | 0,03 mM | 4.0 |
| 13 | ohne Säure | | |
| 14 | Längenstandard: pBR 322/Bst NI | | |

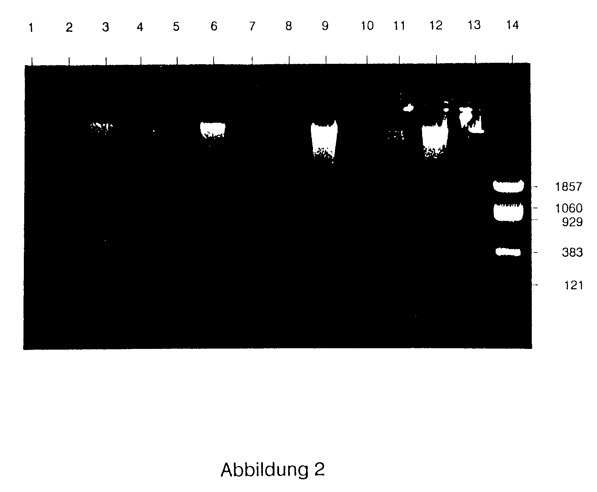

Die Ergebnisse werden in Abbildung 2 dargestellt. Es zeigte sich, daß nach erfindungsgemässer Behandlung der Lambda-Phagen DNA (Lanes 1, 2, 4, 5, 7, 8, 10 und 11) eine deutliche Fragmentierung der DNA nachweisbar ist.

BEISPIEL 4

Behandlung von rekombinanten CHO-Zellen mit Säure

Aus einer Suspensionskultur rekombinanter CHO-Zellen ($3 \times 10^6$ Zellen/ml; Vitalität 96%) wurden Aliquots mit je $1 \times 10^7$ Zellen entnommen und Zitronensäure zugegeben. Das Endvolumen betrug 1 ml. Die Konzentrationen an Zitronensäure waren: [mM] 400, 200, 100, 50, 20, 10. Eine Probe wurde zur Kontrolle mit Wasser vermischt. Es erfolgte eine Inkubation bei 70°C für 60 Minuten (s. Tabelle 4).

Anschliessend wurden die Ansätze nach Zugabe von IM Tris pH 8.5 unter Einstellung des pH-Wertes auf 7 60 Minuten mit Proteinase K (Endkonzentration: 0,4 mg/ml) inkubiert und mit Phenol/Chloroform (1 : 1) zweimal extrahiert. Der wässrige Überstand wurde über eine Sephadex-G25-Säule entsalzt, 90-fach eingeengt und 10 $\mu$l, entsprechend 200 $\mu$l der Suspensionskultur, mit der Agarosegel-Elektrophorese (1,8% Agarose) untersucht.

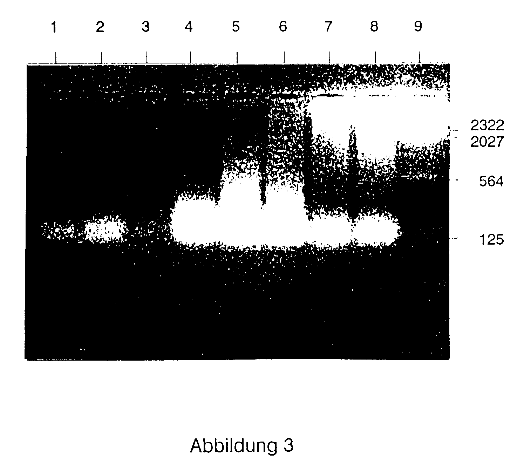

Die Ergebnisse sind in Abbildung 3 dargestellt. Es zeigte sich, daß nach erfindungsgemässer Behandlung der vitalen CHO-Zellen (Lanes 1 bis 6) eine starke Denaturierung und der Abbau der DNA bis zu Fragmenten mit ungefähr 125 bp (Lanes 1-4) nachweisbar ist.

Tabelle 4

| Lane | | | pH der Gesamtlösung |
|---|---|---|---|
| 1 | CHO-Zellen | 400 mM Zitronensäure 70°C/60 Min. | 2.1 |
| 2 | CHO-Zellen | 200 mM Zitronensäure 70°C/60 Min. | 2.3 |
| 3. | CHO-Zellen | 100 mM Zitronensäure 70°C/60 Min. | 2.6 |
| 4 | CHO-Zellen | 50 mM Zitronensäure 70°C/60 Min. | 3.0 |
| 5 | CHO-Zellen | 20 mM Zitronensäure 70°C/60 Min. | 3.7 |
| 6 | CHO-Zellen | 10 mM Zitronensäure 70°C/60 Min. | 4.2 |
| 7 | CHO-Zellen | ohne Zitronensäure 70°C/60 Min. | 6.6 |
| 8 | CHO-Zellen | unbehandelte Zellen | |
| 9 | Längenstandard Lambda DNA/Hind III | | |

BEISPIEL 5

Behandlung von E. Coli (Stamm HB 101) und Bacillus subtilis (ATCC 6633)

Je 1 ml einer Bakterienkultur wurden mit je 1 ml Zitronensäure steigender Konzentration vermischt, wobei folgende Endkonzentrationen an Zitronensäure eingestellt wurden: 0,2 M; 0,4 M; 0,8 M; 1,0 M. Als Kontrolle wurde zu einer Probe statt Zitronensäure 1 ml Wasser gegeben (s. Tabelle 5).

Alle Proben wurden 1 Stunde bei 70°C inkubiert. Anschliessend wurden die Proben für 5 Minuten bei 12.000 xg zentrifugiert und das Sediment mit Proteinase K (Endkonzentration 100 $\mu$g/ml) 1 Stunde bei 37°C verdaut um die Zellmembran aufzuschliessen und gebundene DNA freizusetzen.

Zur Abtrennung von Membranresten, die bei der weiteren Analyse stören, wurde den Proben Kochsalz (Endkonzentration: 0,7 M) und Cetyltrimethyl-ammoniumbromid (Serva, Heidelberg, Best. Nr. 16530) (Endkonzentration: 1%) zugesetzt, 10 Minuten bei 65°C inkubiert und anschliessend mit 1/1 Volumen Chloroform/Isoamylalkohol (24 : 1) extrahiert. Die wässrige Phase, DNA enthaltend, wird durch Extraktion mit Phenol/Chloroform/Isoamylalkohol (25 : 24 : 1) gereinigt, der wässrige Überstand mit Hilfe der Agarosegel-Elektrophorese untersucht. Zur Elektrophorese wurden 10 $\mu$l der aufgereinigten DNA-Lösung, entsprechend 0,4 ml der Kulturbrühe, verwendet.

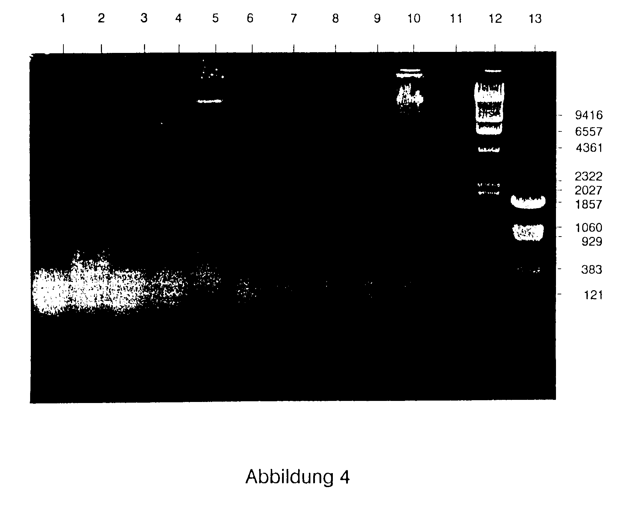

Die Ergebnisse werden in dem Elektropherogramm von Abbildung 4 dargestellt. Es zeigte sich, daß nach der erfindungsgemässen Behandlung der Bakterien nur DNA-Fragmente mit ungefähr 100 bp nachweisbar waren.

EP 0 457 035 B1

Tabelle 5

| Lane | | | pH |
|---|---|---|---|
| 1 | E. coli | 0,2 M Zitronensäure | 1.9 |
| 2 | E. coli | 0,4 M Zitronensäure | 1.8 |
| 3 | E. coli | 0,8 M Zitronensäure | 1.5 |
| 4 | E. coli | 1,0 M Zitronensäure | 1.4 |
| 5 | E. coli | ohne Zitronensäure | |
| 6. | B. subtilis | 0,2 M Zitronensäure | 1.9 |
| 7 | B. subtilis | 0,4 M Zitronensäure | 1.8 |
| 8 | B. subtilis | 0,8 M Zitronensäure | 1.5 |
| 9 | B. subtilis | 1,0 M Zitronensäure | 1.4 |
| 10 | B. subtilis | ohne Zitronensäure | |
| 11 | leer | | |
| 12 | Längenstandard lambda DNA/Hind III | | |
| 13 | Längenstandard pBR 322/Bst NI | | |

**Patentansprüche**

1. Verfahren zur Inaktivierung der biologischen Aktivität der DNA, insbesondere rekombinanter DNA, wobei ein Abbau der DNA erfolgt, dadurch gekennzeichnet, daß die DNA in Anwesenheit einer biologisch abbaubaren Säure ausgewählt aus Ameisensäure, Essigsäure, Propionsäure, Oxalsäure, Malonsäure, Bernsteinsäure, Äpfelsäure, Zitronensäure, Weinsäure, Milchsäure, Maleinsäure und Fumarsäure sowie geeigneter Kombinationen davon bei einer Konzentration von mindestens 0,2 mM und einem pH-Wert von maximal 4 während einer Dauer von 10 bis 70 Minuten auf eine Temperatur von 60 bis 100 °C erhitzt wird.

2. Verfahren gemäß Anspruch 1, dadurch gekennzeichnet, daß die Säure Zitronensäure, Milchsäure, Essigsäure oder Ameisensäure ist.

3. Verfahren gemäß einem der Ansprüche 1 oder 2, dadurch gekennzeichnet, daß die Säurekonzentration 1 mM bis 500 mM beträgt und der pH-Wert zwischen 1 und 3 liegt.

4. Verfahren gemäß einem der Ansprüche 1 oder 2, wobei gegebenenfalls vorhandene Säugetierzellen oder Bakterienzellen gleichzeitig abgetötet werden, dadurch gekennzeichnet, daß zur gleichzeitigen Abtötung von Säugetierzellen eine Säurekonzentration von mindestens 100 mM und ein pH-Wert von maximal 3 und zur gleichzeitigen Abtötung von Bakterienzellen eine Säurekonzentration von mindestens 400 mM und ein pH-Wert von maximal 3 verwendet wird.

**Claims**

1. Process for inactivating the biological activity of DNA, particularly recombinant DNA, in which the DNA is broken down, characterised in that the DNA is heated to a temperature of 60 to 100 °C for a period of 10 to 70 minutes in the presence of a biodegradable acid selected from formic acid, acetic acid, propionic acid, oxalic acid, malonic acid, succinic acid, malic acid, citric acid, tartaric acid, lactic acid, maleic acid and fumaric acid and suitable combinations thereof at a concentration of at least 0.2 mM and at a maximum pH of 4.

2. Process according to claim 1, characterised in that the acid is citric acid, lactic acid, acetic acid or formic acid.

3. Process according to one of claims 1 and 2, characterised in that the acid concentration is 1 mM to 500 mM and the pH is between 1 and 3.

9

4. Process according to one of claims 1 and 2, wherein any mammalian cells or bacterial cells present are simultaneously killed off, characterised in that, in order to kill off mammalian cells simultaneously, an acid concentration of at least 100 mM and a pH of not more than 3 is used and in order to kill off bacterial cells simultaneously an acid concentration of at least 400 mM and a pH of not more than 3 is used.

**Revendications**

1. Procédé pour inactiver l'activité biologique de l'ADN, en particulier de l'ADN recombiné, avec dégradation de l'ADN, caractérisé en ce que l'ADN est chauffé pendant une durée de 10 à 70 minutes à une température de 60 à 100 °C en présence d'un acide biodégradable choisi parmi l'acide formique, l'acide acétique, l'acide propionique, l'acide oxalique, l'acide malonique, l'acide succinique, l'acide malique, l'acide citrique, l'acide tartrique, l'acide lactique, l'acide maléique et l'acide fumarique et de combinaisons appropriées de ceux-ci à une concentration d'au moins 0,2 mM et à un pH d'au plus 4.

2. Procédé selon la revendication 1, caractérisé en ce que l'acide est l'acide citrique, l'acide lactique, l'acide acétique ou l'acide formique.

3. Procédé selon l'une des revendications 1 ou 2, caractérisé en ce que la concentration d'acide est de 1 mM à 500 mM et le pH est situé entre 1 et 3.

4. Procédé selon l'une des revendications 1 ou 2, dans lequel des cellules de mammifère ou des cellules bactériennes éventuellement présentes sont tuées simultanément, caractérisé en ce que, pour tuer simultanément les cellules de mammifère, on utilise une concentration d'acide d'au moins 100 mM et un pH d'au plus 3 et pour tuer simultanément les cellules bactériennes on utilise une concentration d'acide d'au moins 400 mM et un pH d'au plus 3.

1  2  3  4  5  6  7  8  9  10  11  12  13  14

— 1857

— 1060
— 929

— 383

— 121

Abbildung 1

Abbildung 2

Abbildung 3

Abbildung 4